Europäisches Patentamt

European Patent Office

Office européen des brevets

(19)

(11) Numéro de publication : **0 597 749 A1**

# (12) DEMANDE DE BREVET EUROPEEN

(21) Numéro de dépôt : 93402623.8

(22) Date de dépôt : 26.10.93

(51) Int. Cl.$^5$ : **A61F 13/08**

(30) Priorité : **09.11.92 FR 9213475**

(43) Date de publication de la demande :
**18.05.94 Bulletin 94/20**

(84) Etats contractants désignés :
**AT BE CH DE DK ES FR GB GR IE IT LI LU MC
NL PT**

(71) Demandeur : **LABORATOIRES DU PRATICIEN
Rue de la Prairie
F-08430 Poix-Terron (FR)**

(72) Inventeur : **Gobillard, Jean Marie
3 Place de la Mairie
F-08430 Poix-Terron (FR)**
Inventeur : **Muller, Marie Agnès
17 rue de Castrice
F-08000 Charleville Mezières (FR)**
Inventeur : **Bertheas, Alain
23 rue d'Avernay
F-42170 Saint Just Saint Rambert (FR)**
Inventeur : **Ganzoni, Stéfan
Kirschbaumweg 46
CH-4103 Bottmingen (CH)**

(74) Mandataire : **Rodhain, Claude et al
Cabinet Claude Rodhain 30, rue la Boétie
F-75008 Paris (FR)**

(54) **Bande de contention a étalonnage multiple de tension.**

(57) L'invention concerne un article de contention, par exemple une bande élastique (1) comportant un dessin répétitif imprimé (2, 2', 2") dont chacun comporte une pluralité d'éléments.

Ces éléments constituent des formes géométriques ayant des degrés de déformations différents, de façon à ce que plusieurs degrés d'étirement de la bande ramènent l'un après l'autre des éléments (a - e) à une configuration symétrique de cette forme géométrique, ce qui permet de mesurer visuellement le degré d'étirement de la bande, en repérant celui des éléments (a - e) qui prend sa configuration symétrique.

La connaissance du degré de l'étirement de la bande, et en conséquence de la force élastique appliquée par la bande sur le volume à contenir, doit être adaptée à la circonférence du volume à contenir afin de produire la valeur désirée de la pression que la bande exerce sur ce volume.

FIG.1

EP 0 597 749 A1

La présente invention concerne un article de contention comportant une nappe élastique destinée à être positionnée autour d'un volume à contenir, ladite nappe en position autour de ce volume étant étirée afin d'exercer une pression sur ce volume due aux forces élastiques de la nappe étirée, ladite nappe portant des marquages répétitifs dans le sens de l'étirement sur au moins l'une de ses surfaces de façon à ce que le marquage se déforme lors de l'étirement de la nappe.

La demande de brevet français 2 544 982 décrit un article de contention en tissu élastique en forme de bande qui comporte deux rangées de points qui sont imprimés sur la bande, rangées qui se tendent dans le sens longitudinal de la bande.

La distance entre les points d'une rangée est plus courte que la distance entre les deux rangées, de sorte qu'un étirement quelconque de la bande produit un allongement de la distance entre deux points voisins de la même rangée.

Lorsque la distance entre deux points voisins de la même rangée atteint une valeur correspondant à la distance entre les deux rangées qui reste essentiellement invariable durant l'étirement, ceci est une indication pour l'utilisateur que la limite d'étirement permise, sans produire une déformation permanente de la bande, est atteinte.

Le brevet américain 3 613 679 décrit également une bande élastique comportant des marquages en forme de figures géométriques qui se déforment lors de l'étirement de la bande et dont, par exemple, la déformation d'un rectangle en carré signifie un certain degré d'étirement.

La demande de brevet français 2 484 330 décrit une bande comportant un marquage en forme d'ellipse, qui se déforme en cercle lors d'un certain degré d'étirement.

La demande de brevet français 2 666 222 décrit une bande élastique comportant des marquages en forme de rectangle qui se déforment en carré suite à un étirement prédéterminé.

Les bandes de contention qu'on rencontre dans l'art antérieur ont toutes en commun le fait qu'elles permettent de visualiser un seul degré d'étirement de la bande, notamment celui sous lequel les marquages ont une forme symétrique.

Lors de l'application d'une bande, par exemple autour de la jambe d'un être humain, il est souhaitable d'obtenir une valeur bien définie de la pression exercée sur la jambe par la bande étirée car l'effet désiré ne peut pas être atteint si la bande n'est pas suffisamment étirée ; et dans le cas contraire, si la bande est trop étirée, on obtient une surpression dangereuse.

Etant donné que la pression exercée par une bande étirée sur la jambe qu'elle entoure dépend de la circonférence de celle-ci, il n'est pas suffisant d'étirer la bande jusqu'à un certain degré prédéterminé, comme cela serait possible avec une bande de l'art antérieur, car le même degré d'étirement produit des pressions différentes sur des jambes de circonférences différentes.

Le but de la présente invention est de proposer une nouvelle bande qui permette à l'utilisateur de reconnaître une pluralité de degrés d'étirement bien définis afin de pouvoir adapter le degré d'étirement de la bande à la circonférence de la jambe à bander.

Selon l'invention, ce but est obtenu par un article de contention, comme mentionné ci-dessus, qui est caractérisé en ce que lesdits marquages sont constitués d'un dessin répétitif dont chacun comporte une pluralité d'éléments qui sont dimensionnés de façon à représenter, dans l'état non-étiré de la nappe, des configurations déformées à des degrés différents de formes géométriques symétriques, de sorte que chaque élément déformé à l'état non-étiré de la nappe, prend sa forme symétrique lors d'un étirement de la nappe à un degré d'étirement prédéterminé pour ledit élément.

De cette façon, il est possible de choisir un degré d'étirement adapté à la circonférence de la jambe que l'on veut entourer par la bande.

La présente invention se réfère également à l'utilisation d'un tel article de contention, utilisation qui est caractérisée en ce que l'on mesure ou estime la circonférence d'un volume à contenir, en ce que l'on détermine le degré d'étirement nécessaire de la nappe afin d'obtenir une valeur désirée de la pression exercée par la nappe appliquée autour dudit volume en un état d'étirement, pression qui sera essentiellement proche de la pression répondant à la formule :

$$P = \frac{2\,\pi\,F}{C} \times n$$

dans laquelle :

P : est la pression en hPa,

F : est la force élastique de la bande étirée en cN/cm,

C : est la circonférence du volume en cm, et

n : est le nombre de spires formées par la nappe autour dudit volume.

Selon une forme d'exécution particulière de la présente invention, la pluralité d'éléments du dessin comporte des ovales, des ellipses, des rectangles, avec des rapports différents entre leurs dimensions orthogonales prises l'une dans le sens de l'étirement et l'autre dans le sens perpendiculaire à ce dernier.

Selon une forme préférée d'exécution de la présente invention, le dessin comporte X éléments et les dimensions de ces éléments, dans le sens de l'étirement, sont calculées de telle façon qu'elles nécessitent un étirement de la nappe de 10, 20, 30, etc ... jusqu'à X fois 10 %, respectivement, pour amener l'un après l'autre des éléments du dessin à leur configuration de symétrie, à savoir un cercle ou un carré.

Selon une autre forme d'exécution avantageuse de la présente invention, les dessins peuvent comporter des éléments qui sont imprimés de différentes couleurs sur la nappe.

Selon une autre forme d'exécution avantageuse de l'invention, au moins une partie des éléments comporte à l'intérieur de ceux-ci des signes, notamment une lettre, permettant d'indiquer des degrés d'étirement différents de la nappe.

Ainsi, une lettre "L" placée dans un étirement qui, une fois étiré correspond à un étirement long de la nappe, indiquera une contention forte, et une lettre "C" placée dans un élément qui, une fois étiré, correspond à un étirement court de la nappe, indiquera une contention légère.

De cette façon, l'utilisateur pourra vérifier en regardant le signe représenté dans l'élément à l'état étiré, quelle force de contention est appliquée sur le membre.

Une contention forte pendant quelques jours pourra ainsi être suivie d'une contention légère, selon l'efficacité et les résultats obtenus.

Selon une autre forme d'exécution avantageuse de l'invention, au moins une partie des éléments comporte à l'intérieur de ceux-ci un nombre indiquant la quantité de compression, exprimée en millimètres de mercure, obtenue en étirant ledit étirement jusqu'à sa forme symétrique.

A titre indicatif, les nombres 15, 20, 25 et 30 représentent respectivement des taux de compression de 15, 20, 25 et 30 mm Hg.

Selon une autre forme d'exécution avantageuse de l'invention, des flèches sont placées dans le sens d'étirement transversal de la nappe, positionnées de façon à ce que les pointes desdites flèches soient dirigées vers l'extrémité du membre.

De cette façon, l'utilisateur pourra vérifier à tout moment, lors de la pose de l'article de contention, que la nappe est bien positionnée par rapport au membre à bander.

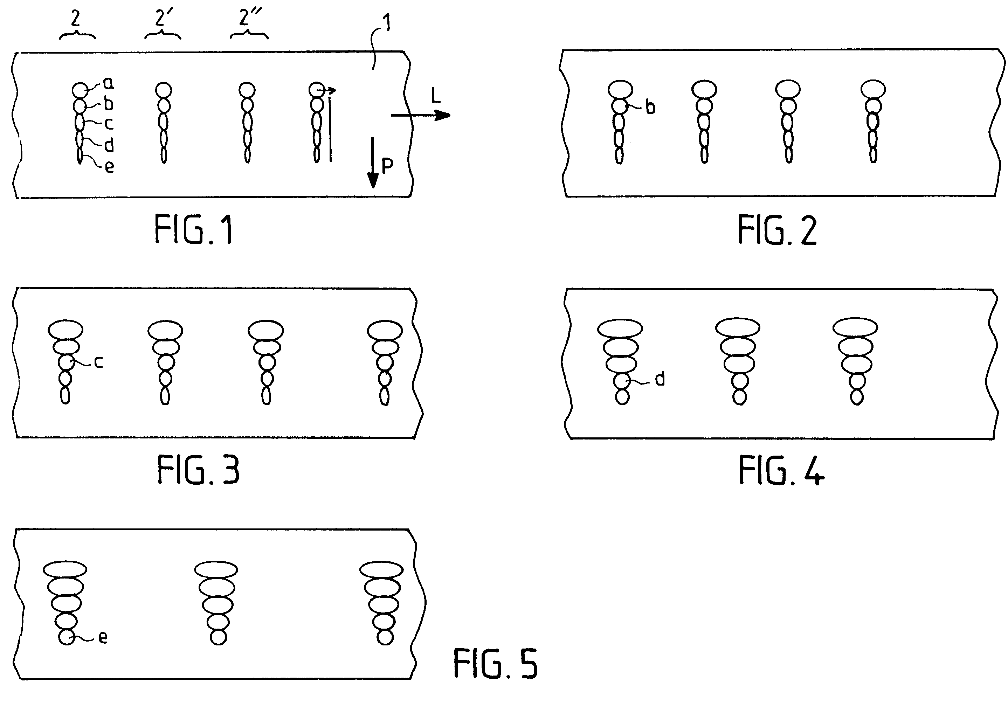

La présente invention sera par la suite décrite plus en détail en se référant aux dessins, dont les figures 1 à 5 montrent des sections de bandes élastiques dans des états d'étirement différents, la figure 1 représentant une bande à l'état non-étiré.

La figure 1 montre une section d'une bande élastique 1 qui comporte des dessins répétitifs 2, 2' dont chacun comporte cinq éléments a, b, c, d et e. Le premier élément a représente un cercle qui correspond à l'état non-étiré de la bande et qui sert à donner une indication de la présence d'une éventuelle hystérèse de la bande après de multiple usages.

La bande 1 de la figure 1 est représentée dans l'état non-étiré et les éléments a ont la forme exacte d'un cercle, ce qui indique que la bande, dans l'état non-étiré, n'a pas subi une déformation permanente.

Les éléments b, c, d et e représentent des ellipses ou des ovales ayant des rapports différents entre leur dimension longitudinale, représentée par la flèche L et leur dimension transversale indiquée par la flèche P, de sorte que la dimension longitudinale diminue en passant de l'élément a à l'élément e.

Lors d'un étirement progressif de la bande 1, on comprend que les éléments a - e se déforment également dans le sens de la flèche 1, et l'un après l'autre, les éléments b - e vont passer à un état où ils prennent la forme d'un cercle exact.

Les dimensions longitudinales des éléments b - e sont calculées de façon à ce que les degrés d'étirement, qui correspondent à la déformation de ces éléments b - e en cercle respectivement, correspondent également à une série de valeurs d'étirement étalonnées, afin de permettre à l'utilisateur de déceler un certain degré d'étirement de la bande en surveillant le développement des éléments b - e jusqu'au moment où l'élément attribué à un certain degré d'étirement obtient sa forme parfaitement ronde.

Comme indiqué également dans la figure 1, le dessin 2 est répétitif en 2', 2" etc ... sur toute la bande afin de permettre le maintien du même degré d'étirement désiré durant toute l'opération d'enroulement de la bande autour d'un certain volume.

Bien évidemment, il est également possible de modifier, de façon contrôlée, le degré d'étirement lors de son application entre les spires individuelles de la bande si on le désire, par exemple lorsque la circonférence de la jambe varie de haut en bas.

Les figures 2 à 5 indiquent plusieurs degrés d'étirement de la même bande et on voit que l'élément b prend une forme parfaitement ronde dans la figure 2, l'élément c est devenu parfaitement rond dans la figure 3, d dans la figure 4 et e dans la figure 5.

A titre d'exemple, les différences des rapports entre les dimensions longitudinale et transversale des différents éléments de chaque dessin 2, 2' etc ... peuvent suivre une série d'étalonnages en intervalles de 10 %, c'est-à-dire que l'étirement, comme indiqué dans la figure 2, ou l'élément b prend une forme parfaitement ronde correspondant à un étirement de 10 %, c'est-à-dire qu'un segment de bande mesurant 10 cm à l'état non étiré est étendu à 11 cm.

Dans les figures 3, 4, et 5 où les éléments c, d et e respectivement prennent leur forme parfaitement ronde, l'étirement de la bande correspond à 20, 30 et 40 % respectivement.

Bien entendu, ces valeurs, formes et dispositions, sont données à titre d'exemple seulement sans limiter l'étendue de la protection de la présente invention.

Les déformations des éléments du dessin dans les figures 2 à 5 ne correspondent évidemment pas exactement au degré d'étirement de 10 à 40 % mentionné ci-dessus, mais sont illustrées de façon exagérée pour mieux comprendre le principe de la présente invention.

Comme mentionné plus haut, il est important d'appliquer une bande de contention, par exemple autour de la jambe d'un être humain, avec un degré d'étirement bien adapté, afin d'obtenir la pression désirée que cette bande exerce sur la jambe, étant entendu qu'un manque de pression ou une pression exagérée pourrait se traduire par un manque de résultat, ou même par une aggravation de la raison pour laquelle on souhaite l'application de la bande autour de la jambe.

La pression exercée par une bande étirée autour d'un volume ayant une certaine circonférence dépend de la force élastique de la bande due à l'étirement et de la circonférence de la bande selon la formule suivante :

$$P = \frac{2\pi F}{C} \times n$$

dans laquelle :

P : est la pression en hPa,

F : est la force élastique de la bande étirée en cN/cm,

C : est la circonférence du volume en cm, et

n : est le nombre de spires formées par la nappe autour dudit volume.

Afin d'obtenir une certaine pression sur la jambe traitée, il est donc important de trouver l'étirement nécessaire pour la circonférence donnée de la jambe, et dans l'application réelle de la bande on va d'abord mesurer ou estimer la circonférence de la jambe afin de trouver la force nécessaire, qui est une fonction linéaire du degré d'étirement de la bande (pour autant que l'étirement reste dans la plage élastique de la bande) et la force ainsi déterminée identifiera un des éléments a - e du dessin de la bande, élément qui va être utilisé lors de l'étirement de la bande pour indiquer le degré d'étirement désiré lorsque cet élément prend sa forme parfaitement ronde.

Durant l'application de la bande autour d'une jambe, par exemple en spires consécutives, on va obtenir un certain chevauchement entre les spires, chevauchement qui peut correspondre par exemple à un quart, un tiers, la moitié, trois quarts de la largeur de la bande et chaque degré de chevauchement produit une certaine moyenne du nombre de spires appliquées sur le même point de la jambe.

Par exemple, un chevauchement de trois quarts produit quatre spires sur chaque point de la jambe (à l'exception des parties extrêmes de l'ensemble de spires), un chevauchement de un sur deux produit un ensemble dans lequel chaque point est couvert par deux spires.

Selon la formule indiquée ci-dessus, le nombre de spires n augmente évidemment linéairement la pression, et le degré de chevauchement des spires doit donc être pris en compte quand on détermine le degré d'étirement (la force F) que l'on doit appliquer pour obtenir la pression P désirée.

Selon une forme particulière de la présente invention, les éléments a - e de chaque dessin 2, 2', etc ... peuvent être imprimés avec une couleur différente, afin de mieux permettre à l'utilisateur de repérer le bon étirement pour un certain cas.

Il est possible de placer les dessins sur une partie de la bande qui sera couverte par la spire suivante afin de rendre le bandage final plus esthétique en cachant les marquages en-dessous des spires suivantes.

A titre d'exemple non limitatif, un marquage à moitié d'une bande selon l'invention, permet avec deux spires d'obtenir la quantité de compression indiquée sur le marquage, en faisant disparaître les dessins et en obtenant ainsi un bandage final esthétique.

Les marquages des bandes avec les dessins 2 sont réalisés avantageusement par impression avec plusieurs têtes d'impression, notamment avec autant de têtes d'impression qu'il y a d'éléments a - e différents dans le cas où ces éléments sont imprimés avec des couleurs différentes.

Bien entendu, il est possible de réaliser cette impression avec une seule tête d'impression lorsque tous les éléments sont imprimés de la même couleur.

L'impression peut être effectuée sur un tapis roulant muni de rouleaux d'entraînement et de distributeurs de couleurs d'impression.

De façon avantageuse, l'impression est réalisée à vitesse lente sur un matériel élastique au repos.

## Revendications

1. Article de contention comportant une nappe élastique (1) destinée à être positionnée autour d'un volume à contenir, ladite nappe mise en position autour de ce volume étant étirée afin d'exercer une pression (P) sur ce volume, due aux forces élastiques (F) de la nappe étirée, ladite nappe portant des marquages (2, 2', 2") répétitifs dans le sens de l'étirement (L) sur au moins l'une de ses surfaces de façon à ce que les marquages se déforment lors de l'étirement de la nappe, caractérisé en ce que lesdits marquages (2, 2', 2") sont constitués d'un dessin répétitif dont chacun comporte une pluralité d'éléments (a - e) qui sont dimensionnés de façon à représenter, dans l'état non-étiré de la nappe, des configurations déformées à des degrés différents de formes géométriques symétriques, de sorte que chaque élément déformé à l'état non-étiré de la nappe, prend sa forme symétrique lors d'un étirement de la nappe à un degré d'étirement prédéterminé pour ledit élément.

2. Article selon la revendication 1, caractérisé en ce que la pluralité d'éléments (a - e) du dessin (2) comporte des ovales, des ellipses, des rectangles à différents rapports entre leurs dimensions orthogonales prises l'une dans le sens de l'étirement (L) et l'autre dans le sens perpendiculaire (P) à ce dernier.

3. Article selon la revendication 2, caractérisé en ce que le dessin comporte X éléments, et en ce que les dimensions de ces éléments (a - e) dans le sens de l'étirement (L) sont calculées de façon qu'elles nécessitent un étirement de la nappe (1) de 10, 20, 30, etc ... jusqu'à X fois 10 % respectivement pour amener l'un après l'autre des éléments du dessin à sa configuration de symétrie, à savoir un cercle ou un carré.

4. Article selon l'une quelconque des revendications 1 à 3, caractérisé en ce qu'au moins une partie des éléments comporte, à l'intérieur de ceux-ci, un signe, notamment une lettre, indiquant le degré d'étirement de la nappe.

5. Article selon l'une quelconque des revendications 1 à 4, caractérisé en ce qu'au moins une partie des éléments comporte, à l'intérieur de ceux-ci, un nombre indiquant le taux de compression, exprimé en mm Hg, obtenu en étirant lesdits éléments dans leur forme symétrique.

6. Article selon l'une quelconque des revendications 1 à 5, caractérisé en ce que la nappe comporte des flèches, positionnées dans le sens d'étirement tranversal de la nappe, les pointes desdites flèches étant dirigées vers l'extrémité du membre à bander.

7. Article selon l'une quelconque des revendications 1 à 6, caractérisé en ce que le dessin (2, 2', 2") comporte des éléments qui sont imprimés avec des couleurs différentes.

8. Article selon l'une quelconque des revendications 1 à 7, caractérisé en ce que l'on mesure ou estime la circonférence d'un volume à contenir, en ce que l'on détermine le degré d'étirement nécessaire de la nappe, afin d'obtenir une valeur désirée de la pression exercée par la nappe appliquée autour dudit volume à l'état d'étirement, ladite pression suivant essentiellement la pression (P) selon la formule :

$$P = \frac{2\pi F}{C} \times n$$

dans laquelle :

P : est la pression en hPa,

F : est la force élastique de la bande étirée en cN/cm,

C : est la circonférence du volume en cm, et

n : est le nombre de spires formées par la nappe autour dudit volume.

FIG.1

FIG.2

FIG.3

FIG.4

FIG.5

EP 0 597 749 A1

Office européen
des brevets

**RAPPORT DE RECHERCHE EUROPEENNE**

Numero de la demande

EP 93 40 2623

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.5) |
|---|---|---|---|
| X | FR-A-2 499 416 (ETABLISSEMENTS THUASNE ET THUASNE-PARIS) <br> * page 3, ligne 18 - page 4, ligne 31 * <br> * figure 1 * <br> --- | 1-5,8 | A61F13/08 |
| X | DE-A-23 29 371 (P.DE VOS) <br> * revendications 1-3,8-11 * <br> * page 5, ligne 1 - ligne 29 * <br> --- | 1,2,7,8 | |
| A | DE-A-36 40 979 (RAUSCHER) <br> * revendications 5-7; figure 2 * <br> --- | 1,3 | |
| D,A | EP-A-0 475 811 (ETABLISSEMENTS THUASNE ET THUASNE-PARIS) <br> * colonne 2, ligne 9 - ligne 17; figures 1-5 * <br> --- | 1,8 | |
| D,A | US-A-3 613 679 (P.W.BIJOU) <br> * colonne 1, ligne 37 - ligne 39 * <br> * colonne 3, ligne 36 - ligne 38 * <br> --- | 4,5,7 | |
| A | EP-A-0 490 793 (MOLINIER) <br> * colonne 2, ligne 6 - ligne 32 * <br> * colonne 2, ligne 50 - ligne 53 * <br> ----- | 6 | DOMAINES TECHNIQUES RECHERCHES (Int.Cl.5) <br><br> A61F |

Le présent rapport a été établi pour toutes les revendications

| Lieu de la recherche | Date d'achèvement de la recherche | Examinateur |
|---|---|---|
| LA HAYE | 14 Janvier 1994 | NICE, P |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

............................................................................

& : membre de la même famille, document correspondant

EPO FORM 1503 03.82 (P04C02)